# EUROPEAN PATENT SPECIFICATION

(11) **EP 1 954 349 B1**
(45) Date of publication and mention of the grant of the patent: **21.12.2011**
(21) Application number: 05804699.6
(22) Date of filing: 23.11.2005
(51) Int. Cl.: A61N 1/368, A61N 1/362

(54) **AN IMPLANTABLE HEART STIMULATION DEVICE**
IMPLANTIERBARE HERZSTIMULATIONSVORRICHTUNG
DISPOSITIF IMPLANTABLE DE STIMULATION CARDIAQUE

(43) Date of publication of application: 13.08.2008
(73) Proprietor: St Jude Medical AB, 17584 Järfälla (SE)
(72) Inventor: BJÖRLING, Anders, S-169 37 Solna (SE)
(86) International application number: PCT/SE2005/001762
(87) International publication number: WO 2007/061344

(56) References cited:
- EP-A- 1 249 254
- EP-A- 1 449 562
- EP-A1- 0 308 535
- EP-A1- 0 536 720
- EP-A2- 0 677 303
- WO-A-01/24874
- WO-A1-91/03274
- US-A- 4 686 989
- US-A1- 2002 183 792
- US-A1- 2002 183 792
- US-B1- 6 192 274
- US-B1- 6 862 477
- GREGORIO DI F ET AL: "Retroconduction selective recognition in wide-dipole floating atrial sensing" PACE - PACING AND CLINICAL ELECTROPHYSIOLOGY, BLACWELL FUTURA PUBLISHING, MALDEN, MA, US, vol. 20, no. 11, 1 November 1997 (1997-11-01), pages 2817-2824, XP000722444 ISSN: 0147-8389
- SOBEL ET AL: "Pacemaker-mediated tachycardia: Management by pacemaker interrogation/reprogramming in the ED" AMERICAN JOURNAL OF EMERGENCY MEDICINE, CENTRUM PHILADELPHIA, PA, US, vol. 20, no. 4, 1 July 2002 (2002-07-01), pages 336-339, XP005700812 ISSN: 0735-6757
- WITTELES ET AL: "Premature ventricular contractions causing pacemaker-mediated tachycardia: A failure of postventricular atrial refractory period after premature ventricular contraction extension?" HEART RHYTHM, ELSEVIER, vol. 2, no. 12, 1 December 2005 (2005-12-01), pages 1389-1390, XP022080881 ISSN: 1547-5271
- GUENOUN M. ET AL.: 'Cross-ventricular pacemaker-mediated tachycardia by myopotential induction during biventricular pacing' PACING AND CLINICAL ELECTROPHYSIOLOGY vol. 28, no. 6, June 2005, pages 585 - 587, XP003001375
- BAROLD S.S. ET AL.: 'Cross-ventricular endless loop tachycardia during biventricular pacing' PACING AND CLINICAL ELECTROPHYSIOLOGY (PACE), US vol. 24, no. 12, December 2001, pages 1821 - 1823, XP003001376

## Description

### BACKGROUND OF THE INVENTION

### 1. Field of the invention

The present invention relates to an implantable heart stimulation device with which it is possible to stimulate both the ventricles of a heart, i.e. a bi-ventricular pacer. The heart stimulation device can be a so-called pacemaker or an ICD (implantable cardioverter defibrillator) which also includes a pacing function.

The invention also relates to a method of, in a patient who is treated with a bi-ventricular cardiac heart stimulation device, detecting a pacemaker-mediated tachycardia of a certain kind.

### 2. Description of the prior art

Several different implantable devices for stimulating a heart are known. The devices are normally able to sense the electrical activity of the heart. Some implantable devices are able to deliver stimulation pulses to and/or sense the right atrium (in some cases even the left atrium) and also to deliver stimulation pulses to and sense both the left and right ventricles.

Devices that are able to deliver stimulation pulses to both the left and right ventricles can be called bi-ventricular pacers. Such devices can be used to treat patients who suffer from different severe cardiac problems, e.g. patients suffering from congestive heart failure (CHF). CHF is defined generally as the inability of the heart to deliver a sufficient amount of blood to the body. CHF can have different causes. It can for example be caused by a left bundle branch block (LBBB) or a right bundle branch block (RBBB). By using bi-ventricular pacing, the contraction of the ventricles can be controlled in order to improve the ability of the heart to pump blood. The stimulation pulses to the two ventricles can be delivered simultaneously but it is also known that the stimulation pulses to the two ventricles are delivered with a short time delay between them in order to optimise the pumping performance of the heart.

US-A-5 720 768 describes different possible electrode positions in order to stimulate or sense the different chambers of the heart.

US-A-6 070 100 describes that electrodes may be positioned in both the left and the right atrium as well as in the left and the right ventricles.

A concept used in connection with heart stimulation devices is pacemaker-mediated tachycardia (PMT). A PMT is a phenomenon that is known in connection with a pacemaker that has both an atrial channel and a ventricular channel. A PMT of this kind is a reentry arrhythmia in which the pulse generator acts as the anterograde limb of the tachycardia and the natural conduction path acts as the retrograde limb. An R-wave (QRS-complex) is thus conducted to the atrium, causing a retrograde P-wave which is sensed by the device. The device then emits a ventricular pacing pulse after a certain delay (PV-delay). This pacing pulse captures the ventricle and the evoked R-wave is then conducted back to the atrium, and the cycle repeats itself. Such an endless loop between an atrium and a ventricle can of course also occur in a heart stimulation device that has two ventricular channels in addition to an atrial channel. US 6,611,714 B 1 discloses a heart stimulation device that deals with such endless loop problems.

The articles "Pacemaker-Mediated Tachycardia in a Biventricular Pacing System" by van Gelder et al., PACE, vol. 24, December 2001, pp. 1819-1820 and "Cross-Ventricular Endless Loop Tachycardia During Biventricular Pacing" by Barold et al., PACE, vol. 24, December 2001, pp. 1821-1823 both describe that an endless loop tachycardia can occur if a dual chamber pacemaker (intended for sensing/pacing in an atrium and in a ventricle) is used as a bi-ventricular device.

EP 1 449 562 A1 describes an implantable bi-ventricular heart stimulating device. The device is able to deliver pacing pulses to both ventricles with a time delay (VV) between the pacing pulses. The device is arranged to operate with particular VV delays (VV_{PVC1} and VV_{PVC2}) in case of a PVC (Premature Ventricular Contraction).

### SUMMARY OF THE INVENTION

The inventor of the present invention has realised that a certain kind of pacemaker-mediated tachycardia can be a problem in a bi-ventricular cardiac heart stimulation device. This pacemaker-mediated tachycardia can be caused by the fact that a sensed event in a first ventricle causes the generation of a pacing pulse to the second ventricle, which pacing pulse causes a depolarisation of the second ventricle with an associated electrical activity, which may also involve a repolarisation, which electrical activity is transferred via the heart tissue to the first ventricle, and, after a certain time, is sensed in the first ventricle, which results in a new pacing pulse being applied to said second ventricle, which placing pulse causes a depolarisation of the second ventricle with an associated electrical activity, which may also involve a repolarisation, which electrical activity is transferred via the heart tissue to the first ventricle, and, after a certain time, is sensed in the first ventricle, and so on.

An object of the present invention is to provide a cardiac heart stimulation device with which it is possible to detect a certain loop of events that if the heart stimulation device were (or is) in operation in a patient could be (or can be) a pacemaker-mediated tachycardia of the above kind.

This object is achieved with a cardiac heart stimulation device including a control circuit comprising:
at least one memory;
a first ventricular sensing circuit, adapted to communicate with a first ventricular sensing electrode suited to be positioned in or at a first ventricle of a heart, wherein said first ventricular sensing circuit is adapted to enable sensing of such a ventricle;
a second ventricular pacing circuit, adapted to communicate with a second ventricular pacing electrode suited to be positioned in or at a second ventricle of a heart, wherein said second ventricular pacing circuit is adapted to enable pacing of such a ventricle;
said control circuit being arranged to be able to operate with time cycles corresponding to normal heart cycles;
said control circuit being arranged to, within a time cycle, be able to sense with the first ventricular sensing circuit and to, after a time gap that is ≥ 0, deliver a pacing pulse with the second ventricular pacing circuit,
the control circuit being arranged to be able to detect the above mentioned loop of events that if the heart stimulation device were in operation in a patient could be a pacemaker-mediated tachycardia of the above mentioned kind,
wherein the control circuit is arranged to perform the following steps:
   detect one or both of the following:
      i) the regularity of one or more repetitious events related to the first ventricular sensing circuit or the second ventricular pacing circuit,
      ii) a repetitive operation pattern of the heart stimulation device,
   determine one or both of the following:
      iii) if the detected regularity fulfils a predetermined regularity criterion,
      iv) if the operation pattern fulfils a predetermined operation pattern criterion, and, based on the above steps; determine whether said loop of events would be likely to be the case if the heart stimulation device were in operation in a patient.

By arranging the control circuit to perform such detection and determination, it is possible to detect whether a PMT of the specific kind defined above is likely to be the case.

Preferably, the heart stimulation device according to the invention is an implantable heart stimulation device, i.e. a device that can be implanted in a human or animal being.

It should be noted that when it is said that for example a certain circuit is adapted to enable sensing and pacing of an atrium or ventricle, this does not necessarily mean that the circuit actually is connected to an atrium or a ventricle. Instead it means that if the heart stimulation device, in which the circuit in question is included, is actually implanted in a body with suitably located electrodes, then the circuit in question would be able to sense and pace an atrium or a ventricle. Similarly, the expressions relating to atrial or ventricular pacing and sensing circuits or the like only mean that these circuits are adapted to be able to sense typical atrial or ventricular events and that they are able to deliver pulses which are of the kind that is typical for stimulating atria or ventricles. A "pacing pulse" or the like is thus a pulse with an energy and morphology which would make it suitable to pace the relevant heart chamber.

It should also be noted that the mentioned "first ventricle" is not necessarily the ventricle that is paced/sensed first, if there is a normal time gap VV between the sense/pace in one ventricle and the pace (which may also be inhibited), during the same time cycle, in the other ventricle. The expressions "first ventricle" and "second ventricle" or the like are thus primarily used in order to distinguish between the two ventricles (or between the two ventricular channels of the heart stimulation device). This fact will be further explained below, when the label VVᵢ is explained.

According to one embodiment of the heart stimulation device according to the invention, the control circuit is arranged such that the detection of said regularity involves detecting the regularity of one or more of the following repetitious events:
a) the time between consecutive sensed events of said first ventricular sensing circuit,
b) the time between consecutive pacing pulses delivered with said second ventricular pacing circuit,
c) the time between a pacing pulse delivered with said second ventricular pacing circuit and the subsequent sensed event of said first ventricular sensing circuit.

Such repetition events can be used in order to determine whether the mentioned PMT is likely to be the case.

According to a further embodiment of the heart stimulation device, the control circuit is arranged such that the regularity criterion is that a measure of how much the respective time varies is below a predetermined value. If the respective time varies very little between different cycles, then it is likely that the mentioned PMT is the case.

According to a further embodiment of the heart stimulation device, the control circuit is arranged to change at least one pacing parameter and determine if this results in a change in the detected regularity and/or in the detected repetitive operation pattern. By changing a pacing parameter and by determining if this results in a corresponding, expected, change in the detected regularity or repetitive operation pattern, it can be determined whether the mentioned PMT is likely to be the case.

The concept "pacing parameter" or the like is meant to cover any setting that influences the operation of the heart stimulation device. Such settings include different programmed times and different sensitivities for detecting signals.

According to a further embodiment of the heart stimulation device, the pacing parameter that is changed is the time between a sensed event of said first ventricular sensing circuit and the subsequent pacing pulse, during the same time cycle, delivered with said second ventricular pacing circuit. Such a change will result in a change in the operation of the heart stimulation device and the change in the operation can then be used to determine whether the mentioned PMT is the case.

According to a further embodiment of the heart stimulation device, the control circuit is arranged to determine a measure related to the rate, or cycle length, with which the heart stimulation device operates before and after the change of the pacing parameter and to determine the time between a pacing pulse delivered with said second ventricular pacing circuit and the subsequent event sensed by said first ventricular sensing circuit before and after the change of the pacing parameter, and based on this information to determine whether said loop of events would be likely to be the case if the heart stimulation device were in operation in a patient. Such determinations can be used to decide whether the mentioned PMT is likely to be the case. How this can be done will be more closely described further below.

According to a further embodiment of the heart stimulation device, the repetitive operation pattern is V2, R1, V2, R1, V2, R1 ...., where V2 is a pacing pulse delivered with said second ventricular pacing circuit and R1 is a sensed event of said first ventricular sensing circuit. The sensed event R1 can be an R-wave or a T-wave.

That the repetitive operation pattern is V2, R1, V2, R1, V2, R1 ...., preferably means that no other events (in an atrium or a ventricle) than the R1 events are sensed, at least not outside the refractory periods of the heart stimulation device, as long as this pattern occurs.
Another possible repetitive operation pattern is V2, P, R1, V2, P, R1, V2, P, R1,.....

According to a further embodiment of the heart stimulation device, the operation pattern criterion is that the operation pattern lasts longer than a predetermined time or that the number of paced and sensed events in said operation pattern is higher than a predetermined number. If the operation pattern lasts a long time, then it is likely that the mentioned PMT is the case.

Another aspect of the invention concerns providing a device that is actually able to break (terminate) the detected loop of events. In accordance with this aspect of the invention, the control circuit is arranged to break the operation pattern with which the heart stimulation device operates by changing the operation of the heart stimulation device, wherein the change in operation is done in a manner particularly adapted to be likely to stop the particular, above defined, loop of events that would be likely to be the case if the heart stimulation device were in operation in a patient.

According to a further embodiment of the heart stimulation device, the change in operation involves preventing at least one pacing pulse of said second ventricular pacing circuit from being generated.

According to a further embodiment of the heart stimulation device, the control circuit is arranged such that said at least one pacing pulse of said second ventricular pacing circuit is prevented from being generated by one or more of the following measures:
a) simply inhibiting at least one such pacing pulse and thereafter return to the normal operation of the heart stimulation device,
b) changing a refractory period of the operation of the heart stimulation device, such that the heart stimulation device does not react on at least one sensed event of said first ventricular sensing circuit, and thereafter return to the normal operation of the heart stimulation device,
c) changing a blanking period of the operation of the heart stimulation device, such that the heart stimulation device does not sense at least one event that would otherwise be sensed by said first ventricular sensing circuit, and thereafter return to the normal operation of the heart stimulation device,
d) changing a sensitivity setting of the operation of the heart stimulation device, such that the heart stimulation device does not sense at least one event that would otherwise be sensed by said first ventricular sensing circuit, and thereafter return to the normal operation of the heart stimulation device with the normal sensitivity setting,
e) delivering a pacing pulse at least substantially simultaneously with a first ventricular pacing circuit, which is comprised in the control circuit, and said second ventricular pacing circuit.

It will be explained further below how these different manners can be used to break the detected loop of events.

According to a further embodiment of the heart stimulation device, the change in operation involves temporarily shortening the pacing parameter which is the time between an event sensed by said first ventricular sensing circuit and the subsequent pacing pulse, during the same time cycle, delivered with said second ventricular pacing circuit, and thereafter return to the normal operation of the heart stimulation device. By shortening the mentioned pacing parameter, it is less likely that the electrical activity caused by a pacing pulse to the second ventricle will be transferred and sensed in the first ventricle. Consequently, it can be possible to break the operation pattern in this manner.

Another aspect of the invention concerns providing a device that is actually able to prevent future occurrences of the loop of events of the above defined kind. In accordance with this aspect of the invention, the control circuit is also arranged to be able to prevent such future occurrences of the loop of events if the heart stimulation device is in operation in a patient, by changing one or more pacing parameters, wherein the change in pacing parameters is done in a manner particularly adapted to prevent the mentioned loop of events.

According to a further embodiment of the heart stimulation device, the control circuit is arranged to store in the memory a sequence of heart stimulation device events, such that, based on an analysis of this sequence of heart stimulation device events, it would be possible to determine the cause said loop of events. By analysing the sequence of events, it is thus possible to determine the likely cause of the endless loop with which the heart stimulation device operates. The sequence of heart stimulation device events can for example be analysed by a physician during a medical check-up.

It can be noted that the expression "heart stimulation device events" includes both sensed events and delivered pacing pulses.

According to a further embodiment of the heart stimulation device, the control circuit is also arranged to carry out an analysis of the stored sequence of heart stimulation device events, and based on this analysis, to automatically change said one or more pacing parameters, such that a future loop of events of the above defined kind is prevented if the heart stimulation device is in operation in a patient. According to this alternative, the device itself thus automatically carries out the necessary change in pacing parameters in order to prevent future occurrences of the endless loop of events.

According to a further embodiment of the heart stimulation device, the control circuit is arranged such that the change in pacing parameters in order to prevent a future loop of events of the above defined kind involves one or more of the following measures:
a) changing at least one sensitivity setting of the operation of the heart stimulation device, such that the heart stimulation device is less sensitive, and thus less likely to sense at least one kind of event, the sensing of which could trigger the above defined loop of events if the heart stimulation device is in operation in a patient,
b) changing at least one sensitivity setting of the operation of the heart stimulation device, such that the heart stimulation device is more sensitive, and thus more likely to sense at least one kind of event, the lack of sensing of which could trigger the above defined loop of events if the heart stimulation device is in operation in a patient,
c) changing the energy of the pacing pulses delivered by the heart stimulation device,
d) extending at least one blanking or refractory period of the operation of the heart stimulation device, such that the heart stimulation device is less likely to react on at least one kind of event, which, if the heart stimulation device reacted on it, could trigger the above defined loop of events if the heart stimulation device is in operation in a patient.

It will be explained further below how these measures may prevent future occurrences of the loop of events.

According to a further embodiment of the heart stimulation device, the control circuit also comprises a first atrial sensing and/or pacing circuit, adapted to communicate with a first atrial sensing and/or pacing electrode suited to be positioned in an atrium of a heart, wherein said first atrial sensing and/or pacing circuit is adapted to enable sensing and/or pacing of such an atrium. The present invention is advantageous to use in connection with a heart stimulation device that also has the ability to sense and/or pace at least one atrium.

Another object of the invention is to provide a method of, in a patient who is treated with a bi-ventricular cardiac heart stimulation device, detecting a pacemaker-mediated tachycardia of the above defined kind.

This object of the invention is achieved by a method that includes the following steps:
detect one or both of the following:
   i) the regularity of one or more repetitious events related to the ventricles,
   ii) a repetitive operation pattern of the heart stimulation device,
determine one or both of the following:
   iii) if the detected regularity fulfils a predetermined regularity criterion,
   iv) if the operation pattern fulfils a predetermined operation pattern criterion, and, based on the above steps, decide whether the above defined pacemaker-mediated tachycardia is likely to be the case.

The method of the invention also concerns breaking a pacemaker-mediated tachycardia of the above defined kind and preventing future occurrences of such a pacemaker-mediated tachycardia. Different manners of carrying out the method of the invention are clear from the claims below.

The method of the invention has advantages corresponding to those described above in connection with the device according to the invention.

Further aspects and advantages of the present invention will become clear from the following description and claims.

### BRIEF DESCRIPTION OF THE DRAWINGS

- Fig 1: shows schematically a heart stimulation system with a heart stimulation device connected to leads with sensing and pacing electrodes positioned in a heart.
- Fig 2: shows schematically a control circuit which may form part of the device.
- Fig 3: shows schematically on a time scale certain events that may occur in heart stimulation device.
- Fig 4: illustrates schematically a method according to the invention.

### DESCRIPTION OF PREFERRED EMBODIMENTS

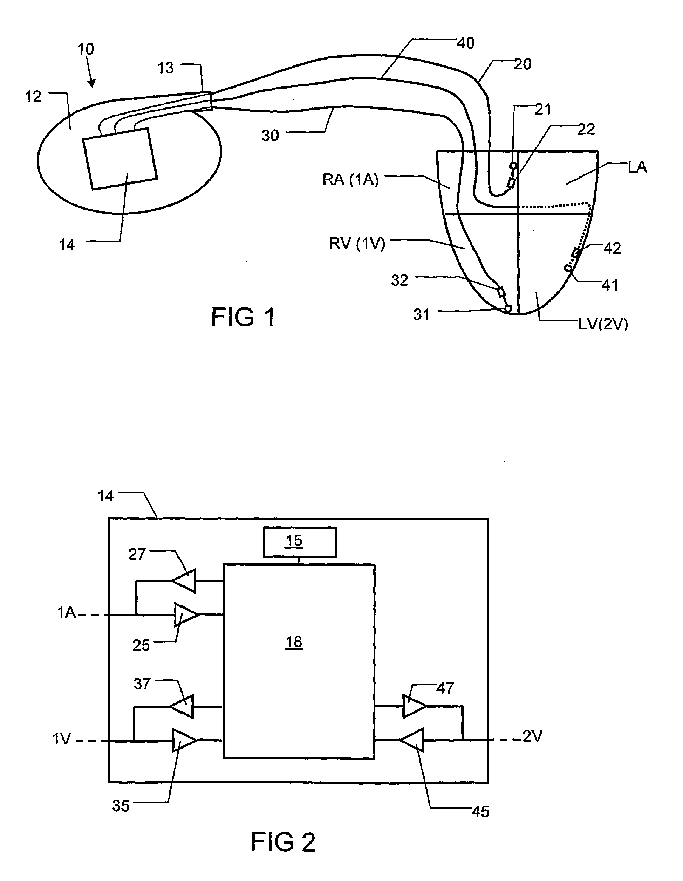

Fig 1 shows schematically an embodiment of an implantable heart stimulation device 10 according to the invention. The device 10 comprises a housing 12. The housing 12 includes a control circuit 14. The device 10 comprises a connector portion 13. Via the connector portion 13, the device 10 can be connected to different leads. In Fig 1 the device 10 is connected to three leads 20, 30 and 40.

The lead 20 includes a pacing and sensing electrode 21, 22. In the shown example, this electrode 21, 22 is a bipolar electrode with a tip portion 21 and a ring portion 22. However, it is within of the scope of the invention that instead unipolar electrodes can be used, as is known to a person skilled in the art. Similarly to the lead 20, the lead 30 includes a pacing and sensing electrode 31, 32 and the lead 40 includes a pacing and sensing electrode 41, 42. The device 10 together with the leads 20, 30, 40 and the electrodes 21, 22; 31, 32; 41 42 can be said to constitute an implantable heart stimulation system.

Fig 1 also schematically illustrates a heart with a right atrium RA, a left atrium LA, a right ventricle RV and a left ventricle LV.

The electrode 21, 22 constitutes a first atrial sensing and/or pacing electrode 21, 22 which is positioned in a first atrium 1A of the heart, according to this embodiment the right atrium RA, in order to enable sensing and/or pacing of this atrium RA.

The electrode 31, 32 constitutes a first ventricular sensing and pacing electrode 31, 32, which is positioned in a first ventricle 1V of the heart, in this embodiment the right ventricle RV. The first ventricular sensing and pacing electrode 31, 32 is adapted to enable sensing and pacing of this first ventricle 1V.

The electrode 41, 42 constitutes a second ventricular sensing and pacing electrode 41, 42, which is positioned at a second ventricle 2V of the heart, in this embodiment the left ventricle LV. The second ventricular sensing and pacing electrode 41, 42 is adapted to enable sensing and pacing of this second ventricle 2V. The lead 40 may for example be introduced via the right atrium RA and the coronary sinus such that the electrode 41, 42 is positioned in for example the middle or great cardiac vein of the heart. How to introduce the lead 40 in this manner is known to a person skilled in the art.

Although not shown in Fig 1, it is also possible that the system is connected to further leads and/or further electrodes, for example electrodes positioned in order to sense and/or pace the left atrium LA and electrodes designed to enable defibrillation.

Fig 2 shows schematically the control circuit 14 in some more detail. The control circuit 14 includes a memory 15 connected to a control portion 18. The control circuit 14 includes a first atrial sensing and/or pacing circuit 25, 27. In this embodiment, this circuit 25, 27 includes a sensing circuit 25 and a pacing circuit 27. The first atrial sensing and/or pacing circuit 25, 27 communicates with the first atrial sensing and/or pacing electrode 21, 22 via the lead 20. The first atrial sensing and/or pacing circuit 25, 27 is thus adapted to sense and/or pace an atrium 1A, in this case the right atrium RA.

The control circuit 14 also includes a first ventricular sensing circuit 35 and a first ventricular pacing circuit 37. These circuits 35, 37 communicate with the first ventricular sensing and pacing electrode 31, 32 via the lead 30. The circuits 35, 37 are thus adapted to sense and pace a first ventricle 1V, in this case the right ventricle RV.

The control circuit 14 also includes a second ventricular sensing circuit 45 and a second ventricular pacing circuit 47. These circuits 45, 47 communicate with the second ventricular sensing and pacing electrode 41, 42 via the lead 40. These circuits 45, 47 are adapted to sense and pace a second ventricle 2V, in this case the left ventricle LV.

The control circuit 14 is arranged, or programmed, to include several operational features.

As is normal in a heart stimulation device, the first ventricular sensing circuit 35 and the second ventricular sensing circuit 45 are able to sense events typical for an R-wave (QRS-complex) in the respective ventricle. The ventricular sensing mentioned in this application is thus primarily designed to sense R-waves. However, it should be noted that the ventricular sensing circuits 35, 45 may sometimes also sense a T-wave (ventricular repolarisation). Consequently, the operation of the device 10 may depend not only on whether an R-wave is sensed but also on a sensed T-wave.

As is also normal in a heart stimulation device, the first atrial sensing and/or pacing circuit 25, 27 is arranged to be able to detect events typical for a P-wave.

The control circuit 14 is arranged to be able to operate with time cycles corresponding to normal heart cycles. Such an operation is normal for an implantable heart stimulation device. The time cycles are determined by preset timer intervals which also may depend on detected signals.

The control circuit 14 is also arranged to, within a time cycle, be able to deliver pacing pules with both said first ventricular pacing circuit 37 and said second ventricular pacing circuit 47 with a time gap VV, during the normal operation of the device 10, between a pacing pulse delivered, or inhibited, by one of said first 37 and second 47 ventricular pacing circuits and a pacing pulse delivered, or inhibited, by the other one of said first 37 and second 47 ventricular pacing circuits, wherein said time gap VV is ≥ 0. A typical value ofVV can be between 0ms and 80ms, for example 30ms. For example, if the sense/pace channel 35, 37 is the channel that is normally first with regard to the VV time gap, then, if the first ventricular sensing circuit 35 senses an event, then a pacing pulse can be delivered with the second ventricular pacing circuit 47 after the time VV. The delivery of the pacing pulse with the second ventricular pacing circuit 47 can also be inhibited if the second ventricular sensing circuit 45 senses an event before the pacing pulse is delivered with the second ventricular pacing circuit 47.

It should also be noted that the control circuit 14 can be arranged to operate in the following manner: if an event is sensed by the ventricular sensing circuit (for example the second ventricular sensing circuit 45) that is associated with the ventricle that is normally paced last (i.e. after the time gap VV) during a time cycle when no event has been sensed by the other ventricular sensing circuit (in this example the first ventricular sensing circuit 35) and no pacing pulse has been delivered with the corresponding ventricular pacing circuit (in this example the first ventricular pacing circuit 37), then a pacing pulse is immediately, or at least almost immediately, delivered with this pacing circuit (in this example thus the first ventricular pacing circuit 37). In order to distinguish between the normal time gap VV explained above and the short delay "immediately, or at least almost immediately" that has just been explained, herein the label VVᵢ is used for the latter. VVᵢ is ≥ 0, but normally quite short, for example 10ms. The time gap that is ≥ 0 that is mentioned in, inter alia, claim 1 below can thus either be the time gap VV or the time gap VVᵢ.

As is normal for implantable heart stimulation devices, the device 10 is also normally set up to operate with PV and AV delays. PV can for example be defined as the time between the sensing with said first atrial sensing and/or pacing circuit 25, 27 and a subsequent pacing pulse, which may also be inhibited, of said first ventricular pacing circuit 37. AV can for example be defined as the time between the pacing with said first atrial sensing and/or pacing circuit 25, 27 and a subsequent pacing pulse, which may also be inhibited, of said first ventricular pacing circuit 37. It is well known to a person skilled in the art how an implantable heart stimulation device is set up in order to operate with PV and AV delays. Furthermore, the device 10 is normally set up to operate with well known blanking and refractory periods.

Although not described in any detail here, the control circuit 14 can be arranged to include several other operational features that are known in connection with heart stimulation devices. Such features include, for example, the ability to detect evoked reponses to delivered pacing pulses (such detection is normally done with a detection logic that is different from that used for detecting R-waves or T-waves); the ability to deliver back-up pulses if a heart chamber is not captured when a pacing pulse has been delivered; the ability to perform capture threshold searches; the ability to sense the physiological activity of the patient in whom the device has been implanted; the ability to carry out defibrillation; the ability to communicate with the help of so-called telemetry, etc.

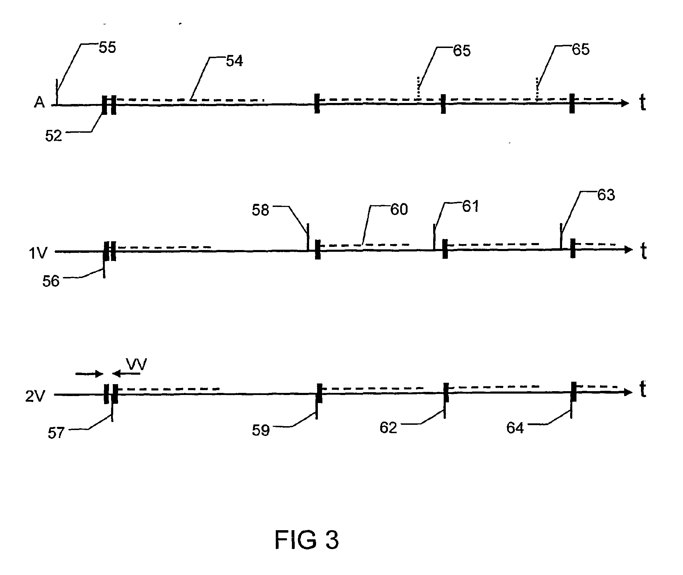

Fig. 3 shows schematically on a common time scale heart stimulation device events related to an atrial channel A, a first ventricular channel 1V and a second ventricular channel 2V. The black markers (like the mark 52) indicate a blanking period for the respective channel. During the blanking period, the sense circuit in question is disabled and no sensing is thus possible during the blanking periods. The hatched lines (like the line 54) indicate refractory periods for the respective channels. The heart stimulation device will not react on possible events that are sensed during such refractory periods. A line pointing upwards (for example the lines 55 and 58) indicates a sensed event in the respective channel and a line pointing downwards (like the lines 56 and 57) indicates a pacing pulse delivered by the respective channel.

Fig. 3 illustrates a chain of events that may occur in a bi-ventricular heart stimulation device. 55 indicates an event sensed by the first atrial sensing circuit 25. After a certain delay (AV-delay) a pacing pulse 56 is delivered with the first ventricular pacing circuit 37. After the time VV a pacing pulse 57 is delivered with the second ventricular pacing circuit 47. A certain sensed event (or the lack of sensing of a certain event that should have been sensed) may cause the heart stimulation device 10 to operate in an endless loop. Fig. 3 illustrates that the first ventricular sensing circuit 35 senses an event 58. This sensing can for example constitute oversensing, i. e. the sensing is not caused by any real cardiac event that should have been sensed. This sensed event 58 causes a pacing pulse 59 to be delivered with the second ventricular pacing circuit 47 after a time gap VV. The pacing pulse 59 causes depolarisation of the second ventricle 2V with an associated electrical activity. This electrical activity may also involve a repolarisation (T-wave). The electrical activity is transferred via the heart tissue to the first ventricle 1V. This transfer takes a certain time. Since the transfer takes a certain time it can "arrive" in the first ventricle 1V after the refractory period 60. This transferred electrical activity is sensed as an event 61 by the first ventricular sensing circuit 35. The event 61 causes the generation of a pacing pulse 62 with the second ventricular pacing circuit 47 after the time gap VV. Because of the mentioned transfer time and the VV time gap, the pacing pulse 62 is emitted to the second ventricle 2V after the biological refractory period caused by the previous pacing pulse 59. The second ventricle 2V is therefore captured again by the pacing pulse 62. Consequently, the second ventricle 2V is depolarised and an associated electrical activity, which may also involve a repolarisation, is transferred via the heart tissue to the first ventricle 1V and is there sensed again as an event 63. The sensed event 63 causes another pacing pulse 64 to be generated by the second ventricular pacing circuit 47. An endless loop of events is thus the case, i. e. a PMT has been generated.

It can be noted that the events 65 and 66 in Fig. 3 illustrate retrograde P-waves occurring in the atrial channel. However, these events 65, 66 do not cause any change in the operation of the heart stimulation device 10, since these events 65, 66 fall within the refractory period for the atrial channel (post ventricular atrial refractory period, PVARP).

It can be noted that although not shown in Fig. 3, it is possible that the first ventricle 1V is in fact the ventricle that is normally associated with the ventricular channel that is the last channel with regard to the VV time gap. A sensing in such a channel can cause an almost immediate (after the time gap VVᵢ) generation of a pacing pulse with the other ventricular channel. This pacing pulse may cause a depolarisation with an associated electrical activity in the ventricle in question. This electrical activity is transferred via the heart tissue to the other ventricle and a similar endless loop of events to that described in connection with Fig. 3 may occur.

According to the present invention, the control circuit 14 is arranged to be able to detect whether the above described loop of events is likely to be the case by performing the following steps:
detect one or both of the following:
   i) the regularity of one or more repetitious events related to the first ventricular sensing circuit 35 or the second ventricular pacing circuit 47,
   ii) a repetitive operation pattern of the heart stimulation device 10,
determine one or both of the following:
   iii) if the detected regularity fulfils a predetermined regularity criterion,
   iv) if the operation pattern fulfils a predetermined operation pattern criterion, and, based on the above steps, determine whether said loop of events would be likely to be the case if the heart stimulation device 10 were in operation in a patient.

The mentioned regularity can be one or more of the following repetitious events:
a) the time between consecutive sensed events of said first ventricular sensing circuit 35,
b) the time between consecutive pacing pulses delivered with said second ventricular pacing circuit 47,
c) the time between a pacing pulse delivered with said second ventricular pacing circuit 47 and the subsequent sensed event of said first ventricular sensing circuit 35.

The regularity criterion can be that a measure of how much the respective time varies is below a predetermined value. If a PMT of the described kind is the case, the time between the respective repetitious event will be very stable. The regularity criterion can thus be that the standard deviation or the coefficient of variance (standard deviation divided by the average) is small for the respective time. In order to determine the standard deviation or the coefficient of variance, the times between the respective consecutive events should be monitored over several time cycles, for example over at least 8 time cycles. It is not necessary to determine the regularity of all the mentioned repetitious events a), b) and c). However if more than one of these repetitious events are monitored, then the accuracy in the determination of whether a PMT of the above-mentioned kind is the case will increase.

The mentioned repetitive operation pattern to be detected can be the repetitive operation pattern V2, R1, V2, R1, V2, R1...., where V2 is a pacing pulse delivered with said second ventricular pacing circuit 47 and R1 is a sensed event of said first ventricular sensing circuit 35. The operation pattern criterion can thereby be that the operation pattern lasts longer than a predetermined time, for example longer than 8 s, or that the number of paced and sensed events in said operation pattern is higher than a predetermined number, for example higher than 10.

In order to further improve the detection of whether the above described loop of events is likely to be the case, the control circuit 14 can be arranged to change at least one pacing parameter and determine if this results in a change in the detected regularity and/or in the detected repetitive operation pattern. The pacing parameter that is changed can be the time VV or VVᵢ between a sensed event of said first ventricular sensing circuit 35 and the subsequent pacing pulse, during the same time cycle, delivered with said second ventricular pacing circuit 47. The control circuit 14 can thereby be arranged to determine a measure related to the rate, or cycle length, with which the heart stimulation device 10 operates before and after the change of the pacing parameter and to determine the time between a pacing pulse delivered with said second ventricular pacing circuit 47 and the subsequent event sensed by said first ventricular sensing circuit 35 before and after the change of the pacing parameter, and based on this information to determine whether said loop of events would be likely to be the case if the heart stimulation device 10 were in operation in a patient. If for example the time VV is increased with 20 ms, then, if the mentioned PMT is the case, also the time between consecutive sensed events (like 61 and 63), or the time between consecutive paced events (like 62 and 64) will increase with 20 ms. However, the time between a paced event (like 62) and the following sensed event (like the event 63) will not change.

It should be noted that in order to increase the accuracy of the detection of the PMT, the different described manners of detecting such a PMT may be combined in one and the same heart stimulation device 10.

Once the above described loop of events has been detected, the control circuit 14 can also be arranged to break, or terminate, the operation pattern with which the heart stimulation device 10 operates by changing the operation of the heart stimulation device 10. The change in operation is done in a manner particularly adapted to be likely to stop the particular, above defined, loop of events that would be likely to be the case if the heart stimulation device 10 were in operation in a patient. The change in operation can involve preventing at least one pacing pulse of said second ventricular pacing circuit 47 from being generated. This can be done by one or more of the following measures:
a) inhibiting at least one such pacing pulse and thereafter return to the normal operation of the heart stimulation device 10,
b) changing a refractory period of the operation of the heart stimulation device 10, such that the heart stimulation device 10 does not react on at least one sensed event of said first ventricular sensing circuit 35, and thereafter return to the normal operation of the heart stimulation device 10,
c) changing a blanking period of the operation of the heart stimulation device 10, such that the heart stimulation device 10 does not sense at least one event that would otherwise be sensed by said first ventricular sensing circuit 35, and thereafter return to the normal operation of the heart stimulation device 10,
d) changing a sensitivity setting of the operation of the heart stimulation device 10, such that the heart stimulation device 10 does not sense at least one event that would otherwise be sensed by said first ventricular sensing circuit 35, and thereafter return to the normal operation of the heart stimulation device 10 with the normal sensitivity setting,
e) delivering a pacing pulse at least substantially simultaneously with a first ventricular pacing circuit 37, which is comprised in the control circuit 14, and said second ventricular pacing circuit 47.

The different points a) to e) will now be explained.
a): If a pacing pulse is not delivered by the second ventricular pacing circuit 47, then no depolarisation will occur in the second ventricle, which means that no electrical activity will be transferred to the first ventricle 1V. Consequently, the PMT is broken.
b): The ventricular refractory period 60 of the first ventricular sensing circuit 35 may be increased such that the heart stimulation device 10 will not react on the sensed event (like 61) transferred from the second ventricle 2V.
c): Similarly, if a blanking period is extended for such a long time that a transferred event is not sensed, then the PMT will be broken.
d): If the first ventricular sensing circuit 35 is made less sensitive, a transferred electrical activity (like the event 61) will not be sensed by the first ventricular sensing circuit 35. Consequently, the PMT will be broken.
e): If pacing pulses are delivered simultaneously by the first ventricular pacing circuit 37 and the second ventricular pacing circuit 47, both the ventricles 2V, 1V will be biologically refractory after the delivery of such pacing pulses. Consequently, it is less likely that a transferred electrical activity will depolarise the first ventricle 1V. If the first ventricle is not depolarised, no event (like the event 61) will occur and be sensed by the first ventricular sensing circuit 35. This will lead to the fact that no pacing pulse (like the pacing pulse 62) will be delivered by the second ventricular pacing circuit 47. The pacing pulse 62 will thus be inhibited, and the PMT will be broken.

Another possible manner of changing the operation of the device 10 in order try to break the mentioned loop of events involves arranging the control circuit 14 to temporarily shorten the pacing parameter VV (or VVᵢ) which is the time between an event sensed by said first ventricular sensing circuit 35 and the subsequent pacing pulse, during the same time cycle, delivered with said second ventricular pacing circuit 47, and thereafter return to the normal operation of the heart stimulation device 10. If VV is made shorter, then it is more likely that the first ventricle 1V is biologically refractory when the electrical activity is transferred from the second ventricle 2V. Consequently, it is less likely that such a transferred electrical activity will depolarise the first ventricle 1V. Therefore, the PMT can be broken.

The control circuit 14 can also be arranged to be able to prevent, or aid in preventing, future occurrences of the loop of events of the above defined kind if the heart stimulation device 10 is in operation in a patient. This can be done by changing one or more pacing parameters, wherein the change in pacing parameters is done in a manner particularly adapted to prevent the mentioned loop of events. The control circuit 14 can hereby be arranged to store in the memory 15 a sequence of heart stimulation device events, such that, based on an analysis of this sequence of heart stimulation device events, it would be possible to determine the cause said loop of events. This analysis can be performed by a physician at a medical check-up, when, for example, via telemetry the physician is informed of the sequence of heart stimulation device events that have been stored in the memory 15. Based on this analysis, the physician can reprogram the device 10 such that future occurrences of the loop of events will be prevented. Alternatively, the control circuit 14 can be arranged to automatically carry out an analysis of the stored sequence of heart stimulation device events, and based on this analysis, to automatically change one or more pacing parameters, such that a future loop of events of the above defined kind is prevented if the heart stimulation device 10 is in operation in a patient.

The control circuit 14 can be arranged such that the change in pacing parameters in order to prevent a future loop of events of the above defined kind involves one or more of the following measures:
a) changing at least one sensitivity setting of the operation of the heart stimulation device 10, such that the heart stimulation device 10 is less sensitive, and thus less likely to sense at least one kind of event, the sensing of which could trigger the above defined loop of events if the heart stimulation device 10 is in operation in a patient,
b) changing at least one sensitivity setting of the operation of the heart stimulation device 10, such that the heart stimulation device 10 is more sensitive, and thus more likely to sense at least one kind of event, the lack of sensing of which could trigger the above defined loop of events if the heart stimulation device 10 is in operation in a patient,
c) changing the energy of the pacing pulses delivered by the heart stimulation device 10,
d) extending at least one blanking or refractory period of the operation of the heart stimulation device 10, such that the heart stimulation device 10 is less likely to react on at least one kind of event, which, if the heart stimulation device 10 reacted on it, could trigger the above defined loop of events if the heart stimulation device 10 is in operation in a patient.

Below follow some explanations concerning these measures a) to d).
a): The PMT can be initiated by oversensing. By making the heart stimulation device 10 (or at least the relevant atrial or ventricular channel of the device 10) less sensitive, it is less likely that oversensing will occur. Consequently, it is less likely that a PMT will be initiated.
b): A PMT can also be initiated by undersensing. If this is the case, then future initiation of the PMT can be prevented by making the heart stimulation device 10 (or the relevant channel of the device 10) more sensitive.
c): A PMT can also be caused by a loss of capture, i. e. by the fact that a certain heart chamber is not captured by a generated pacing pulse. If the energy of the pacing pulses is increased, the probability for a loss of capture decreases. It is, for example, possible for the control circuit 14 to perform a capture threshold test in order to determine an appropriate energy of the pacing pulses such that loss of capture is avoided.
d): By extending blanking or refractory periods, it can be avoided that transferred electrical activity, of the kind explained above, will be sensed by the first ventricular sensing circuit 35. Consequently, it can be avoided that a PMT is initiated.

The invention also concerns a method of, in a patient who is treated with a bi-ventricular cardiac heart stimulation device, detecting a pacemaker-mediated tachycardia of the above defined kind.

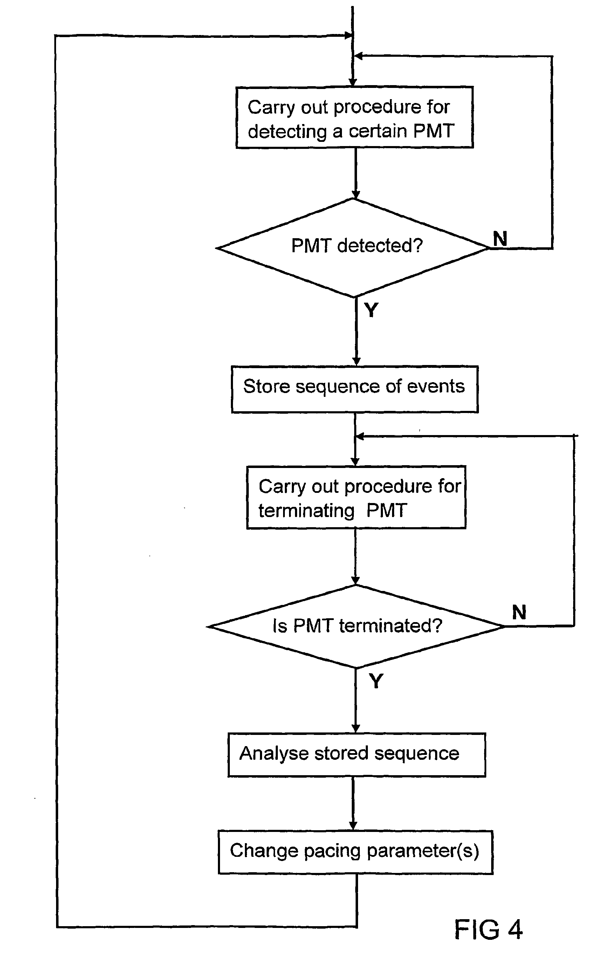

A schematic flow chart illustrating a method according to the invention can be seen in Fig. 4. According to the method, a procedure for detecting a PMT of the above described particular kind is carried out. If a PMT is detected, then the sequence of events that characterises the PMT is stored in a memory. Furthermore, the PMT is terminated. The sequence of events stored in the memory can then be analysed. This can be done either automatically by the device 10 itself or by a physician at a medical check-up. Thereafter, pacing parameters concerning the operation of the heart stimulation device 10 are changed in order to prevent future occurrences of the PMT. The change of pacing parameters can either be performed by a physician or automatically by the device 10 itself.

The method according to the invention will now be described in some more detail. However, since the method in may respects corresponds to the operation of the heart stimulation device 10 explained above, specific details of the method which correspond to the operation of the device are omitted below. The method according to the invention thus includes the following steps:
detect one or both of the following:
   i) the regularity of one or more repetitious events related to the ventricles,
   ii) a repetitive operation pattern of the heart stimulation device,
determine one or both of the following:
   iii) if the detected regularity fulfils a predetermined regularity criterion,
   iv) if the operation pattern fulfils a predetermined operation pattern criterion, and, based on the above steps, decide whether the above defined pacemaker-mediated tachycardia is likely to be the case.

It should be mentioned that, preferably, the method also includes the step of providing means for sensing and/or pacing in at least a first atrium of the heart.

The detection of said regularity can involve detecting the regularity of one or more of the following repetitious events:
a) the time between consecutive sensed events of said first ventricle,
b) the time between consecutive pacing pulses delivered to said second ventricle,
c) the time between a pacing pulse to said second ventricle and the subsequent sensed event of said first ventricle.

The regularity criterion can be that a measure of how much the respective time varies is below a predetermined value.

Analogously to the above description of the device 10 according to the invention, the repetitive operation pattern can be V2, R1, V2, R1, V2, R1...., where V2 is a pacing pulse to the second ventricle and R1 is a sensed event of said first ventricle. The operation pattern criterion can be that the operation pattern lasts longer than a predetermined time or that the number of paced and sensed events in said operation pattern is higher than a predetermined number.

Furthermore, the method may include the step of changing at least one pacing parameter and determine if this results in a change in the detected regularity and/or in the detected repetitive operation pattern. The pacing parameter that is changed can be the time VV or VVᵢ between a sensed event in the first ventricle and the subsequent pacing pulse, during the same time cycle, to the second ventricle. The method can thereby include the step of determining a measure related to the rate, or cycle length, with which the heart stimulation device operates before and after the change of the pacing parameter and also determining the time between a pacing pulse to the second ventricle and the subsequent sensed event in the first ventricle before and after the change of the pacing parameter. Based on this information, it can be determined whether the above defined pacemaker-mediated tachycardia is (likely to be) the case.

The method can also include steps for breaking, or terminating, the detected, above defined, pacemaker-mediated tachycardia. This can be done by changing the operation of the heart stimulation device, wherein the change in operation is done in a manner particularly adapted to stop the particular, above defined, pacemaker-mediated tachycardia that has been detected. This change in operation can involve preventing at least one pacing pulse to the second ventricle from being generated. This can be done by one or more of the following measures:
a) simply inhibiting at least one such pacing pulse and thereafter return to the normal operation of the heart stimulation device,
b) changing a refractory period of the operation of the heart stimulation device, such that the heart stimulation device does not react on at least one sensed event in the first ventricle, and thereafter return to the normal operation of the heart stimulation device,
c) changing a blanking period of the operation of the heart stimulation device, such that the heart stimulation device does not sense at least one event in the first ventricle, and thereafter return to the normal operation of the heart stimulation device,
d) changing a sensitivity setting of the operation of the heart stimulation device, such that the heart stimulation device does not sense at least one event in the first ventricle, and thereafter return to the normal operation of the heart stimulation device with the normal sensitivity setting,
e) deliver a pacing pulse at least substantially simultaneously to the first and the second ventricle.

Another manner of breaking the loop of events can be to temporarily shorten the pacing parameter VV (or VVᵢ) which is the time between a sensed event in the first ventricle and the subsequent pacing pulse, during the same time cycle, to the second ventricle, and thereafter return to the normal operation of the heart stimulation device.

The method of the invention can also include preventing future pacemaker-mediated tachycardias, of the above defined kind. This can be done by changing one or more pacing parameters, wherein the change in pacing parameters is done in a manner particularly adapted to prevent the particular pacemaker-mediated tachycardia that has been detected. This method can include recording and analysing a sequence of heart stimulation device events, and, based on this analysis, determine the cause of the pacemaker-mediated tachycardia, and change said one or more pacing parameters, based on the analysis, such that future pacemaker-mediated tachycardia of the above defined kind is prevented. The change in pacing parameters in order to prevent future pacemaker-mediated tachycardia of the above defined kind, can involve one or more of the following measures:
a) changing at least one sensitivity setting of the operation of the heart stimulation device, such that the heart stimulation device is less sensitive, and thus less likely to sense at least one kind of event in the heart, the sensing of which could trigger the above defined pacemaker-mediated tachycardia,
b) changing at least one sensitivity setting of the operation of the heart stimulation device, such that the heart stimulation device is more sensitive, and thus more likely to sense at least one kind of event in the heart, the lack of sensing of which could trigger the above defined pacemaker-mediated tachycardia,
c) changing the energy of the pacing pulses delivered by the heart stimulation device,
d) extending at least one blanking or refractory period of the operation of the heart stimulation device, such that the heart stimulation device is less likely to react on at least one kind of event in the heart, which, if the heart stimulation device reacted on it, could trigger the above defmed pacemaker-mediated tachycardia.

The method according to the invention can, for example, be performed on a human or animal being suffering from congestive heart failure, for example on a on a human or animal being suffering from a bundle branch block.

The invention is not limited to the described embodiments but may be varied and modified within the scope of the following claims.

## Claims

1. A cardiac heart stimulation device (10) including a control circuit (14) comprising:
at least one memory (15);
a first ventricular sensing circuit (35), adapted to communicate with a first ventricular sensing electrode (31, 32) suited to be positioned in or at a first ventricle (1V) of a heart, wherein said first ventricular sensing circuit (35) is adapted to enable sensing of such a ventricle (1V);
a second ventricular pacing circuit (47), adapted to communicate with a second ventricular pacing electrode (41, 42) suited to be positioned in or at a second ventricle (2V) of a heart, wherein said second ventricular pacing circuit (47) is adapted to enable pacing of such a ventricle (2V);
said control circuit (14) being arranged to be able to operate with time cycles corresponding to normal heart cycles;
said control circuit (14) being arranged to, within a time cycle, be able to sense with the first ventricular sensing circuit (35) and to, after a time gap (VV, VVᵢ) that is ≥ 0, deliver a pacing pulse with the second ventricular pacing circuit (47),
wherein the control circuit (14) also comprises a first atrial sensing and/or pacing circuit (25, 27), adapted to communicate with a first atrial sensing and/or pacing electrode (21, 22) suited to be positioned in an atrium (1 A) of a heart, wherein said first atrial sensing and/or pacing circuit (25, 27) is adapted to enable sensing and/or pacing of such an atrium (1A),
**characterized in that** the control circuit (14) is arranged to be able to detect a pacemaker-mediated tachycardia caused by the fact that a sensed event in a first ventricle causes the generation of a pacing pulse to the second ventricle,
which pacing pulse causes a depolarisation of the second ventricle with an associated electrical activity, which electrical activity is transferred via the heart tissue to the first ventricle, and, after a certain time, is sensed in the first ventricle, which results in a new pacing pulse being applied to said second ventricle, which pacing pulse causes a depolarisation of the second ventricle with an associated electrical activity, which electrical activity is transferred via the heart tissue to the first ventricle, and, after a certain time, is sensed in the first ventricle, and so on,
wherein the control circuit (14) is arranged to be able to perform the following steps:
detect one or both of the following:
i) the regularity with respect to time of one or more repetitious events concerning sensing by the first ventricular sensing circuit (35) and/or pacing by the second ventricular pacing circuit (47),
ii) a repetitive operation pattern concerning sensing and/or pacing of the heart stimulation device (10),
determine one or both of the following:
iii) if the detected regularity fulfils a predetermined regularity criterion,
iv) if the operation pattern fulfils a predetermined operation pattern criterion, and,
based on the above steps, determine whether said pacemaker-mediated tachycardia is likely to be the case.

2. A heart stimulation device (10) according to claim 1, wherein the control circuit (14) is arranged such that the detection of said regularity involves detecting the regularity of one or more of the following repetitious events:
a) the time between consecutive sensed events of said first ventricular sensing circuit (35),
b) the time between consecutive pacing pulses delivered with said second ventricular pacing circuit (47),
c) the time between a pacing pulse delivered with said second ventricular pacing circuit (47) and the subsequent sensed event of said first ventricular sensing circuit (35).

3. A heart stimulation device (10) according to claim 2, wherein the control circuit (14) is arranged such that the regularity criterion is that a measure of how much the respective time varies is below a predetermined value.

4. A heart stimulation device (10) according to any one of the preceding claims, wherein the control circuit (14) is arranged to change at least one pacing parameter and determine if this results in a change in the detected regularity and/or in the detected repetitive operation pattern.

5. A heart stimulation device (10) according to claim 4, wherein the pacing parameter that is changed is the time between a sensed event of said first ventricular sensing circuit (35) and the subsequent pacing pulse, during the same time cycle, delivered with said second ventricular pacing circuit (47).

6. A heart stimulation device (10) according to claim 4 or 5, wherein the control circuit (14) is arranged to determine a measure related to the rate, or cycle length, with which the heart stimulation device (10) operates before and after the change of the pacing parameter and to determine the time between a pacing pulse delivered with said second ventricular pacing circuit (47) and the subsequent event sensed by said first ventricular sensing circuit (35) before and after the change of the pacing parameter, and based on this information to determine whether said pacemaker-mediated tachycardia would be likely to be the case if the heart stimulation device (10) were in operation in a patient

7. A heart stimulation device (10) according to any one of the preceding claims, wherein the repetitive operation pattern is V2, R1, V2, R1, V2, R1...., where V2 is a pacing pulse delivered with said second ventricular pacing circuit (47) and R1 is a sensed event of said first ventricular sensing circuit (35).

8. A heart stimulation device (10) according to claim 7, wherein the operation pattern criterion is that the operation pattern lasts longer than a predetermined time or that the number of paced and sensed events in said operation pattern is higher than a predetermined number.

9. A heart stimulation device (10) according to any one of the preceding claims, wherein the control circuit (14) is arranged to break the operation pattern with which the heart stimulation device (10) operates by changing the operation of the heart stimulation device (10), wherein the change in operation is done in a manner particularly adapted to be likely to stop the particular, above defined, pacemaker-mediated tachycardia that would be likely to be the case if the heart stimulation device (10) were in operation in a patient.

10. A heart stimulation device (10) according to claim 9, wherein the change in operation involves preventing at least one pacing pulse of said second ventricular pacing circuit (47) from being generated.

11. A heart stimulation device (10) according to claim 10, wherein the control circuit (14) is arranged such that said at least one pacing pulse of said second ventricular pacing circuit (47) is prevented from being generated by one or more of the following measures:
a) simply inhibiting at least one such pacing pulse and thereafter return to the normal operation of the heart stimulation device (10),
b) changing a refractory period of the operation of the heart stimulation device (10), such that the heart stimulation device (10) does not react on at least one sensed event of said first ventricular sensing circuit (35), and thereafter return to the normal operation of the heart stimulation device (10),
c) changing a blanking period of the operation of the heart stimulation device (10), such that the heart stimulation device (10) does not sense at least one event that would otherwise be sensed by said first ventricular sensing circuit (35), and thereafter return to the normal operation of the heart stimulation device (10),
d) changing a sensitivity setting of the operation of the heart stimulation device (10), such that the heart stimulation device (10) does not sense at least one event that would otherwise be sensed by said first ventricular sensing circuit (35), and thereafter return to the normal operation of the heart stimulation device (10) with the normal sensitivity setting,
e) delivering a pacing pulse at least substantially simultaneously with a first ventricular pacing circuit (37), which is comprised in the control circuit (14), and said second ventricular pacing circuit (47).

12. A heart stimulation device (10) according to any of the claims 9-11, wherein the change in operation involves temporarily shortening the pacing parameter which is the time between an event sensed by said first ventricular sensing circuit (35) and the subsequent pacing pulse, during the same time cycle, delivered with said second ventricular pacing circuit (47), and thereafter return to the normal operation of the heart stimulation device (10).

13. A heart stimulation device (10) according to any one of the preceding claims, wherein the control circuit (14) is also arranged to be able to prevent future occurrences of the pacemaker-mediated tachycardia of the above defined kind if the heart stimulation device (10) is in operation in a patient, by changing one or more pacing parameters, wherein the change in pacing parameters is done in a manner particularly adapted to prevent the mentioned pacemaker-mediated tachycardia.

14. A heart stimulation device (10) according to claim 13, wherein the control circuit (14) is arranged to store in the memory (15) a sequence of heart stimulation device events, such that, based on an analysis of this sequence of heart stimulation device events, it would be possible to determine the cause of said pacemaker-mediated tachycardia.

15. A heart stimulation device (10) according to claim 14, wherein the control circuit (14) is also arranged to carry out an analysis of the stored sequence of heart stimulation device events, and based on this analysis, to automatically change said one or more pacing parameters, such that a future pacemaker-mediated tachycardia of the above defined kind is prevented if the heart stimulation device (10) is in operation in a patient.

16. A heart stimulation device (10) according to any one claims 13-15, wherein the control circuit (14) is arranged such that the change in pacing parameters in order to prevent a future pacemaker-mediated tachycardia of the above defined kind involves one or more of the following measures:
a) changing at least one sensitivity setting of the operation of the heart stimulation device (10), such that the heart stimulation device (10) is less sensitive, and thus less likely to sense at least one kind of event, the sensing of which could trigger the above defined pacemaker-mediated tachycardia if the heart stimulation device (10) is in operation in a patient,
b) changing at least one sensitivity setting of the operation of the heart stimulation device (10), such that the heart stimulation device (10) is more sensitive, and thus more likely to sense at least one kind of event, the lack of sensing of which could trigger the above defined pacemaker-mediated tachycardia if the heart stimulation device (10) is in operation in a patient,
c) changing the energy of the pacing pulses delivered by the heart stimulation device (10),
d) extending at least one blanking or refractory period of the operation of the heart stimulation device (10), such that the heart stimulation device (10) is less likely to react on at least one kind of event, which, if the heart stimulation device (10) reacted on it, could trigger the above defined pacemaker-mediated tachycardia if the heart stimulation device (10) is in operation in a patient.

## Patentansprüche

1. Herzstimulationsvorrichtung (10) mit einer Steuerungsschaltung, welche das Folgende umfasst:
mindestens einen Speicher (15);
eine erste ventrikuläre Abtastschaltung (35), welche dafür geeignet ist, mit einer ersten ventrikulären Abtastelektrode (31, 32) Daten auszutauschen, die dafür geeignet ist, in oder an einer ersten Herzkammer (1V) angeordnet zu werden, wobei die erste ventrikuläre Abtastschaltung (35) dafür eingerichtet ist, das Abtasten einer solchen Kammer (1V) zu ermöglichen;
eine zweite ventrikuläre Schrittmacherschaltung (47), welche dafür eingerichtet ist, mit einer zweiten ventrikulären Schrittmacherelektrode (41, 42) Daten auszutauschen, die dafür geeignet ist, in oder an einer zweiten Herzkammer (2V) angeordnet zu werden, wobei die zweite ventrikuläre Schrittmacherschaltung (47) dafür eingerichtet ist, die Stimulierung einer solchen Kammer (2V) zu ermöglichen;
wobei die Steuerungsschaltung (14) so eingerichtet ist, dass sie mit Zeitzyklen arbeiten kann, die normalen Herzzyklen entsprechen;
wobei die Steuerungsschaltung (14) so eingerichtet ist, dass sie innerhalb eines Zeitzyklus mit der ersten ventrikulären Abtastschaltung (35) abtasten kann und nach einem Zeitabstand (VV, VVᵢ), der ≥ 0 ist, mit der zweiten ventrikulären Schrittmacherschaltung (47) einen Schrittmacherimpuls übermitteln kann;
wobei die Steuerungsschaltung (14) auch eine erste atriale Abtast- und/oder Schrittmacherschaltung (25, 27) umfasst, welche dafür eingerichtet ist, mit einer ersten atrialen Abtast- und/oder Schrittmacherelektrode (21, 22) Daten auszutauschen, die dafür geeignet ist, in einem Herzvorhof (1A) angeordnet zu werden, wobei die erste atriale Abtast- und/oder Schrittmacherschaltung (25, 27) dafür eingerichtet ist, das Abtasten und/oder die Stimulierung eines solchen Vorhofs (1A) zu ermöglichen;
**dadurch gekennzeichnet, dass** die Steuerungsschaltung (14) dafür eingerichtet ist, eine durch Schrittmacher vermittelte Tachykardie zu erkennen, die von der Tatsache verursacht wird, dass ein erkanntes Ereignis in einer ersten Kammer die Erzeugung eines Schrittmacherimpulses für die zweite Kammer bewirkt, wobei der Schrittmacherimpuls zu einer Depolarisierung der zweiten Kammer mit einer zugehörigen elektrischen Aktivität führt, wobei die elektrische Aktivität über das Herzgewebe in die erste Kammer übertragen wird und nach einer bestimmten Zeit in der ersten Kammer erkannt wird, was dazu führt, dass ein neuer Schrittmacherimpuls an die zweite Kammer angelegt wird, wobei der Schrittmacherimpuls zu einer Depolarisierung der zweiten Kammer mit einer zugehörigen elektrischen Aktivität führt, wobei die elektrische Aktivität über das Herzgewebe in die erste Kammer übertragen wird und nach einer bestimmten Zeit in der ersten Kammer erkannt wird usw.,
wobei die Steuerungsschaltung (14) so eingerichtet ist, dass sie die folgenden Schritte durchführen kann:
Erkennen von einem oder beidem aus dem Folgenden:
i) der zeitlichen Regelmäßigkeit eines oder mehrerer wiederholter Ereignisse betreffend das Abtasten durch die erste ventrikuläre Abtastschaltung (35) und/oder das Stimulieren durch die zweite ventrikuläre Schrittmacherschaltung (47),
ii) einem sich wiederholenden Operationsmuster betreffend das Abtasten und/oder Stimulieren durch die Herzstimulationsvorrichtung (10),
Ermitteln von einem oder beidem aus dem Folgenden:
iii) ob die erkannte Regelmäßigkeit ein vorgegebenes Regelmäßigkeitskriterium erfüllt,
iv) ob das Operationsmuster ein vorgegebenes Operationsmusterkriterium erfüllt, und
Ermitteln auf der Grundlage der obigen Schritte, ob es wahrscheinlich ist, dass die durch Schrittmacher vermittelte Tachykardie vorliegt.

2. Herzstimulationsvorrichtung (10) nach Anspruch 1, wobei die Steuerungsvorrichtung (14) so eingerichtet ist, dass das Erkennen der Regelmäßigkeit das Erkennen der Regelmäßigkeit von einem oder mehreren der folgenden wiederholten Ereignisse umfasst:
a) der Zeit zwischen aufeinander folgenden durch die erste ventrikuläre Abtastschaltung (35) erkannten Ereignissen,
b) der Zeit zwischen aufeinander folgenden Schrittmacherimpulsen, die mit der zweiten ventrikulären Schrittmacherschaltung (47) übermittelt werden,
c) der Zeit zwischen einem Schrittmacherimpuls, der mit der zweiten ventrikulären Schrittmacherschaltung (47) übermittelt wird, und dem darauf folgenden durch die erste ventrikuläre Abtastschaltung (35) erkannten Ereignis.

3. Herzstimulationsvorrichtung (10) nach Anspruch 4, wobei die Steuerungsvorrichtung (14) so eingerichtet ist, dass es sich bei dem Regelmäßigkeitskriterium darum handelt, dass ein Maß, wie oft die entsprechende Zeit variiert, unterhalb eines vorgegebenen Wertes liegt.

4. Herzstimulationsvorrichtung (10) nach einem der vorhergehenden Ansprüche, wobei die Steuerungsschaltung (14) dafür eingerichtet ist, mindestens einen Schrittmacherparameter zu verändern und zu ermitteln, ob dies zu einer Veränderung der erkannten Regelmäßigkeit und/oder des erkannten sich wiederholenden Operationsmusters führt.

5. Herzstimulationsvorrichtung (10) nach Anspruch 4, wobei es sich bei dem Schrittmacherparameter, der verändert wird, um die Zeit zwischen einem durch die erste ventrikuläre Abtastschaltung (35) erkannten Ereignis und dem darauf folgenden Schrittmacherimpuls, der mit der zweiten ventrikulären Schrittmacherschaltung (47) übermittelt wird, während desselben Zeitzyklus' handelt.

6. Herzstimulationsvorrichtung (10) nach Anspruch 4 oder 5, wobei die Steuerungsschaltung (14) dafür eingerichtet ist, ein Maß bezüglich der Geschwindigkeit oder der Zykluslänge, mit welcher die Herzstimulationsvorrichtung (10) arbeitet, vor und nach der Veränderung des Schrittmacherparameters zu ermitteln, und die Zeit zwischen einem Schrittmacherimpuls, der mit der zweiten ventrikulären Schrittmacherschaltung (47) übermittelt wird, und dem darauf folgenden durch die erste ventrikuläre Abtastschaltung (35) erkannten Ereignis vor und nach der Veränderung des Schrittmacherparameters zu ermitteln, und auf der Grundlage dieser Informationen zu ermitteln, ob die durch Schrittmacher vermittelte Tachykardie vorläge, wenn die Herzstimulationsvorrichtung (10) in einem Patienten in Betrieb wäre.

7. Herzstimulationsvorrichtung (10) nach einem der vorhergehenden Ansprüche, wobei es sich bei dem sich wiederholenden Operationsmuster um V2, R1, V2, R1, V2, R1... handelt, wobei V2 für einen Schrittmacherimpuls steht, der mit der zweiten ventrikulären Schrittmacherschaltung (47) übermittelt wird, und R1 für ein durch die erste ventrikuläre Abtastschaltung (35) erkanntes Ereignis steht.

8. Herzstimulationsvorrichtung (10) nach Anspruch 7, wobei es sich bei dem Operationsmusterkriterium darum handelt, dass das Operationsmuster länger als eine vorgegebene Zeit anhält oder dass die Anzahl der stimulierten und erkannten Ereignisse in dem Operationsmuster höher als eine vorgegebene Anzahl ist.

9. Herzstimulationsvorrichtung (10) nach einem der vorhergehenden Ansprüche, wobei die Steuerungsschaltung (14) dafür eingerichtet ist, das Operationsmuster, mit welchem die Herzstimulationsvorrichtung (10) arbeitet, durch Verändern des Betriebs der Herzstimulationsvorrichtung (10) zu durchbrechen, wo bei der Betrieb auf eine Weise verändert wird, die besonders dafür geeignet ist, dass sie wahrscheinlich die spezielle oben definierte, durch Schrittmacher vermittelte Tachykardie beendet, die wahrscheinlich vorläge, wenn die Herzstimulationsvorrichtung (10) in einem Patienten in Betrieb wäre.

10. Herzstimulationsvorrichtung (10) nach Anspruch 9, wobei die Veränderung des Betriebs das Verhindern der Erzeugung mindestens eines Schrittmacherimpulses durch die zweite ventrikuläre Schrittmacherschaltung (47) umfasst.

11. Herzstimulationsvorrichtung (10) nach Anspruch 10, wobei die Steuerungsschaltung (14) dafür eingerichtet ist, dass die Erzeugung des mindestens einen Schrittmacherimpulses durch die zweite ventrikuläre Schrittmacherschaltung (47) durch eine oder mehrere der folgenden Maßnahmen verhindert wird:
a) einfaches Verhindern mindestens eines solchen Schrittmacherimpulses und anschließendes Zurückkehren zum normalen Betrieb der Herzstimulationsvorrichtung (10),
b) Verändern einer Unempfindlichkeitsperiode für den Betrieb der Herzstimulationsvorrichtung (10), derart, dass die Herzstimulationsvorrichtung (10) auf mindestens ein durch die erste ventrikuläre Abtastschaltung (35) erkanntes Ereignis nicht reagiert, und anschließendes Zurückkehren zum normalen Betrieb der Herzstimulationsvorrichtung (10),
c) Verändern einer Blindperiode für den Betrieb der Herzstimulationsvorrichtung (10), derart, dass die Herzstimulationsvorrichtung (10) mindestens ein Ereignis nicht erkennt, welches ansonsten von der ersten ventrikulären Abtastschaltung (35) erkannt werden würde, und anschließendes Zurückkehren zum normalen Betrieb der Herzstimulationsvorrichtung (10),
d) Verändern einer Empfindlichkeitseinstellung für den Betrieb der Herzstimulationsvorrichtung (10), derart, dass die Herzstimulationsvorrichtung (10) mindestens ein Ereignis nicht erkennt, welches ansonsten von der ersten ventrikulären Abtastschaltung (35) erkannt werden würde, und anschließendes Zurückkehren zum normalen Betrieb der Herzstimulationsvorrichtung (10) mit der normalen Empfindlichkeitseinstellung,
e) Übermitteln eines Schrittmacherimpulses zumindest im Wesentlichen gleichzeitig mit einer ersten ventrikulären Schrittmacherschaltung (37), welche in der Steuerungsschaltung (14) enthalten ist, und der zweiten ventrikulären Schrittmacherschaltung (47).

12. Herzstimulationsvorrichtung (10) nach einem der Ansprüche 9 bis 11, wobei die Veränderung des Betriebs das vorübergehende Verkürzen des Schrittmacherparameters umfasst, bei welchem es sich um die Zeit zwischen einem durch die erste ventrikuläre Abtastschaltung (35) erkannten Ereignis und dem darauf folgenden Schrittmacherimpuls, der mit der zweiten ventrikulären Schrittmacherschaltung (47) übermittelt wird, während desselben Zeitzyklus' handelt, und anschließendes Zurückkehren zum normalen Betrieb der Herzstimulationsvorrichtung (10).

13. Herzstimulationsvorrichtung (10) nach einem der vorhergehenden Ansprüche, wobei die Steuerungsschaltung (14) auch so eingerichtet ist, dass sie ein zukünftiges Auftreten der durch Schrittmacher vermittelten Tachykardie der oben definierten Art verhindern kann, wenn die Herzstimulationsvorrichtung (10) in einem Patienten in Betrieb ist, indem ein oder mehrere Schrittmacherparameter verändert werden, wobei die Veränderung der Schrittmacherparameter auf eine Weise erfolgt, die besonders dafür geeignet ist, die erwähnte durch Schrittmacher vermittelte Tachykardie zu verhindern.

14. Herzstimulationsvorrichtung (10) nach Anspruch 13, wobei die Steuerungsschaltung (14) dafür eingerichtet ist, in dem Speicher (15) eine Sequenz von Herzstimulationsvorrichtungsereignissen zu speichern, derart, dass es auf der Grundlage der Analyse dieser Sequenz von Herzstimulationsvorrichtungsereignissen möglich wäre, den Grund für die durch Schrittmacher vermittelte Tachykardie zu ermitteln.

15. Herzstimulationsvorrichtung (10) nach Anspruch 14, wobei die Steuerungsschaltung (14) auch dafür eingerichtet ist, eine Analyse der gespeicherten Sequenz von Herzstimulationsvorrichtungsereignissen vorzunehmen und auf der Grundlage dieser Analyse automatisch den einen oder die mehreren Schrittmacherparameter zu verändern, derart, dass eine zukünftige durch Schrittmacher vermittelte Tachykardie der oben definierten Art verhindert wird, wenn die Herzstimulationsvorrichtung (10) in einem Patienten in Betrieb ist.

16. Herzstimulationsvorrichtung (10) nach einem der Ansprüche 13 bis 15, wobei die Steuerungsschaltung (14) so eingerichtet ist, dass die Veränderung der Schrittmacherparameter, um eine zukünftige durch Schrittmacher vermittelte Tachykardie der oben definierten Art zu verhindern, eine oder mehrere der folgenden Maßnahmen umfasst:
a) Verändern mindestens einer Empfindlichkeitseinstellung für den Betrieb der Herzstimulationsvorrichtung (10), derart, dass die Herzstimulationsvorrichtung (10) weniger empfindlich ist, und dass es für zumindest eine Art eines Ereignisses weniger wahrscheinlich ist, dass die Herzstimulationsvorrichtung (10) es erkennt, wobei die Erkennung desselben die oben definierte durch Schrittmacher vermittelte Tachykardie auslösen könnte, wenn die Herzstimulationsvorrichtung (10) in einem Patienten in Betrieb ist,
b) Verändern mindestens einer Empfindlichkeitseinstellung für den Betrieb der Herzstimulationsvorrichtung (10), derart, dass die Herzstimulationsvorrichtung (10) empfindlicher ist, und dass es für zumindest eine Art eines Ereignisses wahrscheinlicher ist, dass die Herzstimulationsvorrichtung (10) es erkennt, wobei die fehlende Erkennung desselben die oben definierte durch Schrittmacher vermittelte Tachykardie auslösen könnte, wenn die Herzstimulationsvorrichtung (10) in einem Patienten in Betrieb ist,
c) Verändern der Energie des Schrittmacherimpulses, der von der Herzstimulationsvorrichtung (10) übermittelt wird,
d) Verlängern mindestens einer Blind- oder Unempfindlichkeitsperiode für den Betrieb der Herzstimulationsvorrichtung (10), derart, dass es für zumindest eine Art eines Ereignisses weniger wahrscheinlich ist, dass die Herzstimulationsvorrichtung (10) auf dieses reagiert, wobei das Ereignis, wenn die Herzstimulationsvorrichtung (10) auf dieses reagiert, die oben definierte durch Schrittmacher vermittelte Tachykardie auslösen könnte, wenn die Herzstimulationsvorrichtung (10) in einem Patienten in Betrieb ist.

## Revendications

1. Dispositif de stimulation cardiaque (10) comportant un circuit de commande (14) qui comprend :
au moins une mémoire (15) ;
un premier circuit de détection ventriculaire (35) adapté pour communiquer avec une première électrode de détection ventriculaire (31, 32) apte à être positionnée dans ou au niveau d'un premier ventricule (1V) d'un coeur, ledit premier circuit de détection ventriculaire (35) étant adapté pour permettre la détection de ce ventricule (1V) ;
un deuxième circuit de stimulation ventriculaire (47) adapté pour communiquer avec une deuxième électrode de stimulation ventriculaire (41, 42) apte à être positionnée dans ou au niveau d'un deuxième ventricule (2V) d'un coeur, ledit deuxième circuit de stimulation ventriculaire (47) étant adapté pour permettre la stimulation de ce ventricule (2V) ;
ledit circuit de commande (14) étant agencé pour pouvoir fonctionner avec des cycles temporels correspondant à des cycles cardiaques normaux ;
ledit circuit de commande (14) étant agencé pour pouvoir, à l'intérieur d'un cycle temporel, effectuer la détection avec le premier circuit de détection ventriculaire (35) et pour délivrer, après un intervalle de temps (VV, VVᵢ) qui est ≥ 0, une impulsion de stimulation avec le deuxième circuit de stimulation ventriculaire (47),
le circuit de commande (14) comprenant également un premier circuit de détection et/ou stimulation auriculaire (25, 27) adapté pour communiquer avec une première électrode de détection et/ou stimulation auriculaire (21, 22) apte à être positionnée dans une oreillette (1A) d'un coeur, ledit premier circuit de détection et/ou stimulation auriculaire (25, 27) étant adapté pour permettre la détection et/ou la stimulation de cette oreillette (1A),
**caractérisé en ce que** le circuit de commande (14) est agencé pour pouvoir détecter une tachycardie induite par le stimulateur résultant du fait qu'un événement détecté dans un premier ventricule provoque la génération d'une impulsion de stimulation du deuxième ventricule, laquelle impulsion de stimulation provoque une dépolarisation du deuxième ventricule avec une activité électrique associée, laquelle activité électrique est transmise par le biais du tissu cardiaque au premier ventricule et, après un certain temps, détectée dans ledit premier ventricule, ce qui entraîne l'application d'une nouvelle impulsion de stimulation audit deuxième ventricule, laquelle impulsion de stimulation provoque une dépolarisation du deuxième ventricule avec une activité électrique associée, laquelle activité électrique est transmise par le biais du tissu cardiaque audit premier ventricule et, après un certain temps, détectée dans le premier ventricule, et ainsi de suite,
le circuit de commande (14) étant agencé pour pouvoir effectuer les opérations suivantes :
détecter l'une des caractéristiques suivantes ou les deux :
i) la régularité dans le temps d'un ou plusieurs événements répétitifs concernant la détection par le premier circuit de détection ventriculaire (35) et/ou la stimulation par le deuxième circuit de stimulation ventriculaire (47),
ii) un schéma de fonctionnement répétitif concernant la détection et/ou la stimulation du dispositif de stimulation cardiaque (10),
déterminer l'une des caractéristiques suivantes ou les deux :
iii) si la régularité détectée satisfait un critère de régularité prédéterminé,
iv) si le schéma de fonctionnement satisfait un critère de schéma de fonctionnement prédéterminé, et
sur la base des opérations ci-dessus, déterminer s'il est probable que ladite tachycardie induite par le stimulateur est présente.

2. Dispositif de stimulation cardiaque (10) selon la revendication 1, dans lequel le circuit de commande (14) est agencé de telle manière que la détection de ladite régularité comprend la détection de la régularité d'un ou plusieurs des événements répétitifs suivants :
a) le temps entre des événements détectés consécutifs dudit premier circuit de détection ventriculaire (35),
b) le temps entre des impulsions de stimulation consécutives délivrées avec ledit deuxième circuit de stimulation ventriculaire (47),
c) le temps entre une impulsion de stimulation délivrée avec ledit deuxième circuit de stimulation ventriculaire (47) et l'événement détecté suivant dudit premier circuit de détection ventriculaire (35).

3. Dispositif de stimulation cardiaque (10) selon la revendication 2, dans lequel le circuit de commande (14) est agencé de telle manière que le critère de régularité est qu'une mesure du degré de variation du temps respectif se situe en dessous d'une valeur prédéterminée.

4. Dispositif de stimulation cardiaque (10) selon l'une quelconque des revendications précédentes, dans lequel le circuit de commande (14) est agencé pour modifier au moins un paramètre de stimulation et déterminer si ceci entraîne une modification de la régularité détectée et/ou du schéma de fonctionnement répétitif détecté.

5. Dispositif de stimulation cardiaque (10) selon la revendication 4, dans lequel le paramètre de stimulation qui est modifié est le temps entre un événement détecté dudit premier circuit de détection ventriculaire (35) et l'impulsion de stimulation suivante délivrée pendant le même cycle temporel avec ledit deuxième circuit de stimulation ventriculaire (47).

6. Dispositif de stimulation cardiaque (10) selon la revendication 4 ou la revendication 5, dans lequel le circuit de commande (14) est agencé pour déterminer une mesure concernant la fréquence ou la longueur de cycle à laquelle le dispositif de stimulation cardiaque (10) fonctionne avant et après la modification du paramètre de stimulation et pour déterminer le temps entre une impulsion de stimulation délivrée avec ledit deuxième circuit de stimulation ventriculaire (47) et l'événement suivant détecté par ledit premier circuit de détection ventriculaire (35) avant et après la modification du paramètre de stimulation, et pour déterminer sur la base de cette information s'il est probable que ladite tachycardie induite par le stimulateur serait présente si le dispositif de stimulation cardiaque (10) était en fonctionnement chez un patient.

7. Dispositif de stimulation cardiaque (10) selon l'une quelconque des revendications précédentes, dans lequel le schéma de fonctionnement répétitif est V2, R1, V2, R1, V2, R1 ..., où V2 est une impulsion de stimulation délivrée avec ledit deuxième circuit de stimulation ventriculaire (47) et R1 est un événement détecté dudit premier circuit de détection ventriculaire (35).

8. Dispositif de stimulation cardiaque (10) selon la revendication 7, dans lequel le critère de schéma de fonctionnement est que le schéma de fonctionnement dure plus longtemps qu'un temps prédéterminé ou que le nombre d'événements stimulés et détectés dans ledit schéma de fonctionnement est supérieur à un nombre prédéterminé.

9. Dispositif de stimulation cardiaque (10) selon l'une quelconque des revendications précédentes, dans lequel le circuit de commande (14) est agencé pour rompre le schéma de fonctionnement selon lequel le dispositif de stimulation cardiaque (10) opère en modifiant le fonctionnement du dispositif de stimulation cardiaque (10), la modification du fonctionnement se faisant d'une manière particulièrement adaptée pour pouvoir stopper la tachycardie induite par le stimulateur particulière, définie ci-dessus, qui serait probablement présente si le dispositif de stimulation cardiaque (10) était en fonctionnement chez un patient.

10. Dispositif de stimulation cardiaque (10) selon la revendication 9, dans lequel la modification du fonctionnement comprend le fait d'empêcher la génération d'au moins une impulsion de stimulation dudit deuxième circuit de stimulation ventriculaire (47).

11. Dispositif de stimulation cardiaque (10) selon la revendication 10, dans lequel le circuit de commande (14) est agencé de telle manière que la génération de ladite au moins une impulsion de stimulation dudit deuxième circuit de stimulation ventriculaire (47) est empêchée par une ou plusieurs des mesures suivantes :
a) simple inhibition d'au moins une de ces impulsions de stimulation, suivie du retour au fonctionnement normal du dispositif de stimulation cardiaque (10),
b) modification d'une période réfractaire du fonctionnement du dispositif de stimulation cardiaque (10) de telle manière que le dispositif de stimulation cardiaque (10) ne réagisse pas à au moins un événement détecté dudit premier circuit de détection ventriculaire (35), suivie du retour au fonctionnement normal du dispositif de stimulation cardiaque (10),
c) modification d'une période d'occultation du fonctionnement du dispositif de stimulation cardiaque (10) de telle manière que le dispositif de stimulation cardiaque (10) ne détecte pas au moins un événement qui dans le cas contraire serait détecté par ledit premier circuit de détection ventriculaire (35), suivie du retour au fonctionnement normal du dispositif de stimulation cardiaque (10),
d) modification d'un réglage de sensibilité du fonctionnement du dispositif de stimulation cardiaque (10) de telle manière que le dispositif de stimulation cardiaque (10) ne détecte pas au moins un événement qui dans le cas contraire serait détecté par ledit premier circuit de détection ventriculaire (35), suivie du retour au fonctionnement normal du dispositif de stimulation cardiaque (10) avec le réglage de sensibilité normal,
e) délivrance d'une impulsion de stimulation de manière au moins sensiblement simultanée avec un premier circuit de stimulation ventriculaire (37), intégré dans le circuit de commande (14), et ledit deuxième circuit de stimulation ventriculaire (47).

12. Dispositif de stimulation cardiaque (10) selon l'une quelconque des revendications 9 à 11, dans lequel la modification du fonctionnement comprend la réduction temporaire du paramètre de stimulation qui est le temps entre un événement détecté par ledit premier circuit de détection ventriculaire (35) et l'impulsion de stimulation suivante délivrée pendant le même cycle temporel avec ledit deuxième circuit de stimulation ventriculaire (47), suivie du retour au fonctionnement normal du dispositif de stimulation cardiaque (10).

13. Dispositif de stimulation cardiaque (10) selon l'une quelconque des revendications précédentes, dans lequel le circuit de commande (14) est également agencé pour pouvoir empêcher des apparitions futures de la tachycardie induite par le stimulateur du type défini ci-dessus si le dispositif de stimulation cardiaque (10) est en fonctionnement chez un patient, en modifiant un ou plusieurs paramètres de stimulation, la modification des paramètres de stimulation se faisant d'une manière particulièrement adaptée pour empêcher la tachycardie induite par le stimulateur précitée.

14. Dispositif de stimulation cardiaque (10) selon la revendication 13, dans lequel le circuit de commande (14) est agencé pour stocker dans la mémoire (15) une séquence d'événements du dispositif de stimulation cardiaque de façon à pouvoir, sur la base d'une analyse de cette séquence d'événements du dispositif de stimulation cardiaque, déterminer la cause de ladite tachycardie induite par le stimulateur.

15. Dispositif de stimulation cardiaque (10) selon la revendication 14, dans lequel le circuit de commande (14) est également agencé pour effectuer une analyse de la séquence d'événements du dispositif de stimulation cardiaque mémorisés et, sur la base de cette analyse, modifier automatiquement le ou lesdits paramètres de stimulation de manière à empêcher une future tachycardie induite par le stimulateur du type défini ci-dessus si le dispositif de stimulation cardiaque (10) est en fonctionnement chez un patient.

16. Dispositif de stimulation cardiaque (10) selon l'une quelconque des revendications 13 à 15, dans lequel le circuit de commande (14) est agencé de telle manière que la modification des paramètres de stimulation pour empêcher une future tachycardie induite par le stimulateur du type défini ci-dessus comprend une ou plusieurs des mesures suivantes :
a) modification d'au moins un réglage de sensibilité du fonctionnement du dispositif de stimulation cardiaque (10) de manière que le dispositif de stimulation cardiaque (10) soit moins sensible et donc moins susceptible de détecter au moins un type d'événement dont la détection pourrait déclencher la tachycardie induite par le stimulateur définie ci-dessus si le dispositif de stimulation cardiaque (10) est en fonctionnement chez un patient,
b) modification d'au moins un réglage de sensibilité du fonctionnement du dispositif de stimulation cardiaque (10) de manière que le dispositif de stimulation cardiaque (10) soit plus sensible et donc plus susceptible de détecter au moins un type d'événement dont la non-détection pourrait déclencher la tachycardie induite par le stimulateur définie ci-dessus si le dispositif de stimulation cardiaque (10) est en fonctionnement chez un patient,
c) modification de l'énergie des impulsions de stimulation délivrées par le dispositif de stimulation cardiaque (10),
d) allongement d'au moins une période d'occultation ou réfractaire du fonctionnement du dispositif de stimulation cardiaque (10) de manière que le dispositif de stimulation cardiaque (10) soit moins susceptible de réagir à au moins un type d'événement qui, si le dispositif de stimulation cardiaque (10) y réagissait, pourrait déclencher la tachycardie induite par le stimulateur définie ci-dessus si le dispositif de stimulation cardiaque (10) est en fonctionnement chez un patient.
